# EUROPÄISCHE PATENTSCHRIFT

(11) **EP 1 155 740 B1**
(45) Veröffentlichungstag und Bekanntmachung des Hinweises auf die Patenterteilung: **18.10.2006**
(21) Anmeldenummer: 01109287.1
(22) Anmeldetag: 17.04.2001
(51) Int. Cl.: B01J 21/04, B01J 27/122, C07C 17/156, B01J 23/78, B01J 23/83

(54) **Katalysatoren für heterogen katalysierte Reaktionen**
Catalysts for heterogeneously catalysed reactions
Catalyseurs pour réactions catalysées de facon hétérogène

(30) Priorität: 19.05.2000 DE 10024928
(43) Veröffentlichungstag der Anmeldung: 21.11.2001
(73) Patentinhaber: BASF Aktiengesellschaft, 67056 Ludwigshafen (DE)
(72) Erfinder: Walsdorff, Christian, Dr., 67061 Ludwigshafen (DE); Meissner, Ruprecht, 67273 Weisenheim (DE); Harth, Klaus, Dr., 67317 Altleiningen (DE)

(56) Entgegenhaltungen:
- EP-A- 0 278 922
- EP-A- 0 657 212
- EP-A- 0 920 908
- EP-A- 1 053 789
- DE-A- 2 638 498
- US-A- 4 271 042
- US-A- 4 722 920

## Beschreibung

Die vorliegende Erfindung betrifft Katalysatoren für heterogen katalysierte Oxichlorierungsreaktionen mit Aktivkomponenten und einem Katalysatorträger, der röntgendiffraktographisch detektierbare Mengen δ-Al₂O₃ enthält.

Aus der EP-A-375 202 und der US-A-5 011 808 sind Katalysatoren für die Oxichlorierung bekannt, die Kupfer, Kalium und Magnesium auf einem Träger aus ε-Al₂O₃ oder γ-Al₂O₃ enthalten.

Aus der EP-A-931 587 sind Katalysatoren für die Oxichlorierung bekannt, die auf einem γ-Al₂O₃-Träger eine erste Schicht von Magnesium und eine zweite Schicht von Kupfer und wahlweise Lithium enthalten.

Aus der EP-A-255 156 sind Katalysatoren für die Oxichlorierung auf einem γ-Al₂O₃-Träger bekannt, die Kupfer, Magnesium und Natrium oder Lithium enthalten.

Aus der US-A-5 527 754 sind Katalysatoren für die Oxichlorierung bekannt, die Kupfer, Magnesium und Cäsium bzw. eine Mischung von Cäsium und Kalium auf einem Aluminiumoxid enthalten. Als gut geeignet wird γ-Al₂O₃ genannt.

Diese Katalysatoren lassen jedoch noch zu wünschen übrig.

Der vorliegenden Erfindung lag daher die Aufgabe zugrunde, Trägerkatalysatoren für die Oxichlorierung mit verbesserten Eigenschaften bereitzustellen.

Demgemäß wurden neue und verbesserte Katalysatoren für heterogen katalysierte Oxichlorierungst reaktionen gefunden, welche dadurch gekennzeichnet sind, daß der Katalysator als Aktivkomponenten (i) 1 bis 15 Gew.-% Kupfer, (ii) 0,1 bis 6 Gew.-% Alkalimetalle, (iii) 0 bis 5 Gew.-% Erdalkalimetalle, Seltene Erdmetalle oder deren Gemische und einen Katalysatorträger mit röntgendiffraktographisch detektierbaren Mengen δ-Al₂O₃ enthält.

δ-Al₂O₃ ist kommerziell oder durch Calcinierung von Pseudoböhmit bei Temperaturen um ca. 1000°C erhältlich.

Die Katalysatorträger haben in der Regel eine BET-Öberfläche von 80 bis 250 m²/g, bevorzugt von 100 bis 200 m²/g, besonders bevorzugt von 120 bis 180 m²/g und ein Porenvolumen von 0,2 bis 1 cm³/g, bevorzugt von 0,3 bis 0,8 cm³/g, besonders bevorzugt von 0,4 bis 0,7 cm³/g.

δ-Al₂O₃ läßt sich anhand seines Röntgenbeugungsdiagramms zuordnen. In "Aluminium Compounds, G. Mac Zura, K. P. Goodboy und J. J. Koenig, Kirk-Othmer Encyclopedia of Technology Volume 2, Third Edition (1978), Seiten 218 bis 244 ist eine Übersicht mit weiterführenden Referenzen zur Herstellung und Charakterisierung von δ-Al₂O₃ zu finden. δ-Al₂O₃ ist kommerziell erhältlich.

Katalysatorträger mit röntgendiffraktographisch detektierbaren Mengen δ-Al₂O₃ enthalten in der Regel 10 bis 100 Gew.-% δ-Al₂O₃, bevorzugt 30 bis 100 Gew.-% δ-Al₂O₃, besonders bevorzugt 50 bis 100 Gew.-% δ-Al₂O₃, insbesondere 60 bis 100 Gew.-% δ-Al₂O₃. Herstellungsbedingt können noch Restbestandteile von γ-Al₂O₃ vorhanden sein.

Die erfindungsgemäßen Katalysatoren enthalten zusätzlich zum Katalysatorträger Aktivkomponenten. Als Aktivkomponenten eignen sich 1 bis 15 Gew.-%, bevorzugt 2 bis 10 Gew.-%, besonders bevorzugt 4 bis 8 Gew.-% Kupfer, 0,1 bis 6 Gew.-%, bevorzugt 0,2 bis 4 Gew.-%, besonders bevorzugt 0,2 bis 3 Gew.-%, insbesondere 0,25 bis 2 Gew.-% eines Alkalimetalls, wie Lithium, Natrium, Kalium, Cäsium, vorzugsweise Kalium, und gegebenenfalls 0 bis 5 Gew.-%, bevorzugt 0,1 bis 3 Gew.-%, besonders bevorzugt 0,1 bis 2 Gew.-% eines Erdalkalimetalls, wie Calcium, Magnesium, Barium und Strontium, vorzugsweise Magnesium, eines Seltenen Erdmetalles, wie Cer oder Lanthan, oder deren Gemische.

Lösliche Salze sind solche, die sich in Wasser, einem C₁- bis C₄-Alkanol wie Methanol, Ethanol, Propanol oder Butanol, einem Keton wie Aceton oder einem Ester wie Essigsäuremethylester oder Essigsäureethylester, bevorzugt in Wasser lösen.

Als lösliche Salze eignen sich beispielsweise Chloride, Nitrate, Carbonate und Acetate, bevorzugt Chloride, Nitrate und Acetate, besonders bevorzugt Chloride.

Die erfindungsgemäßen Katalysatoren können auch Verunreinigungen aus dem Einsatzstoff des δ-Al₂O₃, dem Pseudoböhmit wie beispielsweise Eisen enthalten.

Die erfindungsgemäßen Katalysatoren werden vorteilhaft durch Tränkung des Katalysatorträgers Trocknen bei 80 bis 250°C, bevorzugt 90 bis 200°C, besonders bevorzugt 100 bis 150°C erhalten. Die Tränkung kann einstufig oder in mehreren Schritten durchgeführt werden. Die Tränkung kann vorzugsweise einstufig und "trocken" durchgeführt werden. "Trocken" bedeutet, daß Konzentration und Menge der Tränklösung so auf die Wasseraufnahme des verwendeten Trägers abgestimmt wird, daß die Tränklösung nahezu vollständig vom Trägermaterial aufgenommen wird und das ganze Trägermaterial gleichmäßig getränkt wird. Gegebenenfalls können auch Säuren wie anorganische Säuren, beispielsweise Salzsäure und Salpetersäure oder organische Säuren wie Carbonsäuren, beispielsweise Essigsäure, bevorzugt Salzsäure oder Oxidationsmittel wie Wasserstoffperoxid zur Tränklösung hinzugefügt werden, um beispielsweise eine klare Tränklösung zu erhalten und die Tränkung zu vereinfachen. Für den Einsatz als Fließbettkatalysatoren wird vorzugsweise ein pulverförmiger Träger getränkt, für den Einsatz als Festbettkatalysator werden vorzugsweise geformte und gegebenenfalls zuvor kalzinierte Formkörper des Trägermaterials eingesetzt.

Die Katalysatoren können in Pulverform für den Einsatz in Fließbettverfahren oder als Formkörper für den Einsatz in Festbettverfahren verwendet werden. Beim Einsatz in Festbettverfahren werden vorzugsweise Träger mit einer druckverlustarmen Geometrie und mit einer hohen geometrischen Oberfläche verwendet, wie beispielsweise Ringe oder Hohlzylinder mit einer oder mehrerer Bohrungen. Um eine bessere mechanische Stabilität oder Porenstruktur solcher Formkörper zu erreichen, kann δ-Al₂O₃ oder auch eine Verbindung eingesetzt werden, die sich nach der Formgebung des Trägerkörpers durch Kalzinieren in δ-Al₂O₃ umwandeln läßt, beispielsweise Pseudoböhmit.

Die erfindungsgemäßen Katalysatoren zeichnen sich gegenüber in herkömmlicher Weise unter Verwendung von γ-Al₂O₃ hergestellten Katalysatoren mit gleichem prozentualem Gehalt an Aktivmasse durch eine deutlich bessere Selektivität bei vergleichbarer Aktivität aus.

Die erfindungsgemäßen Katalysatoren eignen sich für heterogen katalysierte Oxichlorierungsreaktionen, insbesondere zur Oxichlorierung von Ethylen zu 1,2-Dichlorethan (Ethylendichlorid).

Die Oxichlorierung, insbesondere diejenige von Ethylen zu 1,2-Dichlorethan (Ethylendichlorid) läßt sich bei Temperaturen von 150 bis 400°C, bevorzugt von 170 bis 350°C, besonders bevorzugt von 200 bis 300°C und einem Druck von 1 bis 10 bar, bevorzugt von 1 bis 6 bar, besonders bevorzugt von 1 bis 4 bar durchführen.

### Beispiele

Die Katalysatoren wurden in Pulverform in einem Laborfließbettreaktor getestet. Beispiel- und Vergleichskatalysatoren wurden unter den gleichen Bedingungen in derselben Testapparatur getestet. Der Laborreaktor hatte einen Durchmesser von 2,5 cm und wurde durch ein Ölumwälzbad in der Reaktordoppelwand temperiert. Die Temperatur des Wirbelbetts wurde über ein in einer Thermohülse steckendes Thermoelement in der Wirbelschicht gemessen und geregelt. Jeweils 90 g der Katalysatoren wurden mit einem stöchiometrischen Feed von einem Mol Chlorwasserstoff, einem halben Mol Ethylen und einem viertel Mol Sauerstoff in Form von Luft belastet und bei einem Druck von 1,2 bara und Temperaturen von 225°C, 245°C und 265°C getestet. Unter diesen Bedingungen wurde wegen der relativ kurzen Verweilzeit (ca. 7 s) kein Vollumsatz erreicht.

Die erfindungsgemäßen Katalysatoren wurden durch Tränkung von Puralox® SCCa 5/150 (einem δ-Al₂O₃ der Fa. Condea) mit einer klaren Lösung der Promotoren in Wasser erhalten. Die Vergleichskatalysatoren wurde auf dem Träger Puralox® SCCa 5/200 (einem γ-Al₂O₃ der Fa. Condea) hergestellt. Es wurden verschiedene erfindungsgemäße Katalysatoren und Vergleichskatalysatoren jeweils mit gleicher Promotorenzusammensetzung hergestellt und getestet.

### Beispiel 1

34,93 g CuCl₂·2H₂O. 15,84 g MgCl₂·6H₂O und 1,80 g KCl wurden in 156 ml Wasser gelöst. Diese Lösung wurde unter Mischen auf 200 g Puralox® SCCa 5/150 getränkt. Der getränkte Träger wurde 1 h bei Raumtemperatur stehen gelassen und anschließend für 16 h unter einem Stickstoffstrom bei 110°C getrocknet.

**Tab. 1 Testergebnisse des Katalysators aus Beispiel 1**

| Temperatur | Umsatz Ethylen | Selektivität Ethylendichlorid | Selektivität CO + CO₂ | Selektivität Chlorkohlenwasserstoffe* | Ausbeute Ethylendichlorid |
|---|---|---|---|---|---|
| 225°C | 61,1% | 99,6% | 0,14% | 0,17% | 60,9% |
| 245°C | 77,3% | 99,0% | 0,58% | 0,35% | 76,6% |
| 265°C | 82,9% | 96,8% | 2,34% | 0,82% | 80,3% |

| | | | | | |
|---|---|---|---|---|---|
| * Summe von Ethylchlorid, 1,1,2-Trichlorethan, 1,1,2,2-Tetrachlorethan, 1,1-Dichlorethan, Dichlorethen (Isomere), Chloral, Vinylchlorid, Tetrachlorkohlenstoff und Chloroform | | | | | |

### Vergleichsbeispiel 1

34,93 g CuCl₂·2H₂O, 15,84 g MgCl₂·6H₂O und 1,80 g KCl wurden in 160 ml Wasser gelöst. Diese Lösung wurde unter Mischen auf 200 g Puralox® SCCa 5/200 getränkt. Der getränkte Träger wurde 1 h bei Raumtemperatur stehen gelassen und anschließend für 16 h unter einem Stickstoffstrom bei 110°C getrocknet.

**Tab. 2 Testergebnisse des Katalysators aus Vergleichbeispiel 1**

| Temperatur | Umsatz Ethylen | Selektivität Ethylendichlorid | Selektivität CO + CO₂ | Selektivität Chlorkohlenwasserstoffe* | Ausbeute Ethylendichlorid |
|---|---|---|---|---|---|
| 225°C | 65,9% | 99,4% | 0,39% | 0,2% | 65,5% |
| 245°C | 78,5% | 98,3% | 1,18% | 0,44% | 77,2% |
| 265°C | 83,5% | 95,2% | 3,72% | 1,05% | 79,6% |

| | | | | | |
|---|---|---|---|---|---|
| * Summe von Ethylchlorid, 1,1,2-Trichlorethan, 1,1,2,2-Tetrachlorethan, 1,1-Dichlorethan, Dichlorethen (Isomere), Chloral, Vinylchlorid, Tetrachlorkohlenstoff und Chloroform | | | | | |

## Patentansprüche

1. Katalysatoren für heterogen katalysierte Oxichlorierungsreaktionen, **dadurch gekennzeichnet, dass** der Katalysator als Aktivkomponenten
(i) 1 bis 15 Gew.-% Kupfer,
(ii) 0.1 bis 6 Gew.-% Alkalimetalle
(iii) 0 bis 5 Gew.-% Erdalkalimetalle, Seltene Erdmetalle oder deren Gemische
und einen Katalysatorträger mit röntgendiffraktometrisch detektierbaren Mengen δ-Al₂O₃ enthält.

2. Katalysatoren nach Anspruch 1, **dadurch gekennzeichnet, dass** der Katalysatorträger 10 bis 100 Gew.-% δ-Al₂O₃ enthält.

3. Katalysatoren nach einem der Ansprüche 1 oder 2, **dadurch gekennzeichnet, dass** das als Katalysatorträger verwendete δ-Al₂O₃ eine BET-Oberfläche von 80 bis 250 m²/g hat.

4. Katalysatoren nach einem der Ansprüche 1 bis 3, **dadurch gekennzeichnet, dass** das als Katalysatorträger verwendete δ-Al₂O₃ ein Porenvolumen von 0.2 bis 1 cm³/g hat.

5. Verfahren zur Herstellung von Katalysatoren gemäß einem der Ansprüche 1 bis 4, **dadurch gekennzeichnet, dass** man den δ-Al₂O₃ enthaltenden Katalysatorträger mit Salzen von Kupfer, Alkalimetallen und gegebenenfalls Erdalkalimetallen, Seltenen Erdmetallen oder deren Gemischen getrennt voneinander oder gemeinsam, gegebenenfalls unter Zusatz von Säuren und Oxidationsmitteln tränkt.

6. Verfahren zur Herstellung von Katalysatoren nach Anspruch 5, **dadurch gekennzeichnet, dass** man als Salze Chloride einsetzt.

7. Verfahren zur Herstellung von 1,2-Dichlorethan, **dadurch gekennzeichnet, dass** man Ethylen mit Chlorwasserstoff und Luft oder Sauerstoff in Gegenwart eines Katalysators gemäß einem der Ansprüche 1 bis 4 bei einer Temperatur von 150 bis 400°C und bei einem Druck von 1 bis 10 bar umsetzt.

8. Verwendung von Katalysatorenenthaltend als Aktivkomponenten
(i) 1 bis 15 Gew.-% Kupfer,
(ii) 0.1 bis 6 Gew.-% Alkalimetalle
(iii) 0 bis 5 Gew.-% Erdalkalimetalle, Seltene Erdmetalle oder deren Gemische
und einen Katalysatorträger mit röntgendiffraktometrisch detektierbaren Mengen δ-Al₂O₃, für heterogen katalysierte Oxichlorierungsreaktionen.

9. Verwendung von Katalysatoren nach Anspruch 8, **dadurch gekennzeichnet, dass** es sich bei der Oxichlorierungsreaktion um die Umsetzung von Ethylen zu 1,2-Dichlorethan handelt.

## Claims

1. Catalysts for heterogeneously catalyzed oxychlorination reactions, **characterized in that** the catalyst includes as active components
(i) 1 to 15% by weight of copper,
(ii) 0.1 to 6% by weight of alkali metals,
(iii) 0 to 5% by weight of alkaline earth metals, rare-earth metals or mixtures thereof
and a catalyst support comprising amounts of δ-Al₂O₃ which can be detected by X-ray diffractometry.

2. Catalysts according to claim 1, **characterized in that** the catalyst support includes 10 to 100% by weight of δ-Al₂O₃.

3. Catalysts according to either of claims 1 and 2, **characterized in that** the δ-Al₂O₃ used as catalyst support has a BET surface area in the range from 80 to 250 m²/g.

4. Catalysts according to any one of claims 1 to 3, **characterized in that** the δ-Al₂O₃ used as catalyst support has a pore volume in the range from 0.2 to 1 cm³/g.

5. A process for preparing catalysts according to any one of claims 1 to 4, **characterized in that** the δ-Al₂O₃-containing catalyst support is impregnated with salts of copper, alkali metals and, if appropriate, alkaline earth metals, rare-earth metals or mixtures thereof, separately from one another or together, if appropriate with the addition of acids and oxidants.

6. A process for preparing catalysts according to claim 5, **characterized in that** the salts employed are chlorides.

7. A process for preparing 1,2-dichloroethane, **characterized in that** ethylene is reacted with hydrogen chloride and air or oxygen in the presence of a catalyst according to any one of claims 1 to 4 at a temperature in the range from 150 to 400°C and at a pressure in the range from 1 to 10 bar.

8. The use of catalysts including as active components
(i) 1 to 15% by weight of copper,
(ii) 0.1 to 6% by weight of alkali metals,
(iii) 0 to 5% by weight of alkaline earth metals, rare-earth metals or mixtures thereof,
and a catalyst support comprising amounts of δ-Al₂O₃ which can be detected by X-ray diffractometry for heterogeneously catalyzed oxychlorination reactions.

9. The use of catalysts according to claim 8, **characterized in that** the oxychlorination reaction is the conversion of ethylene to 1,2-dichloroethane.

## Revendications

1. Catalyseurs pour réactions d'oxychloration catalysées de façon hétérogène, **caractérisés en ce que** le catalyseur contient, comme composants actifs,
(i) 1 à 15 % en poids de cuivre,
(ii) 0,1 à 6 % en poids de métaux alcalins,
(iii) 0 à 5 % en poids de métaux alcalino-terreux, de métaux des terres rares ou de leurs mélanges,
et un support de catalyseur à quantités de δ-Al₂O₃ détectables par diffractomètre aux rayons X.

2. Catalyseurs suivant la revendication 1, **caractérisés en ce que** le support de catalyseur contient 10 à 100 % en poids de δ-Al₂O₃.

3. Catalyseurs suivant l'une des revendications 1 et 2, **caractérisés en ce que** le δ-Al₂O₃ utilisé comme support de catalyseur présente une surface BET de 80 à 250 m²/g.

4. Catalyseurs suivant l'une des revendications 1 à 3, **caractérisés en ce que** le δ-Al₂O₃ utilisé comme support de catalyseur présente un volume poreux de 0,2 à 1 cm³/g.

5. Procédé de préparation de catalyseurs suivant l'une des revendications 1 à 4, **caractérisé en ce qu'**on imprègne le support de catalyseur contenant du δ-Al₂O₃ par des sels de cuivre, des métaux alcalins et éventuellement des métaux alcalino-terreux, des métaux des terres rares ou leurs mélanges, séparément l'un de l'autre ou conjointement, éventuellement avec addition d'acides et d'oxydants.

6. Procédé de préparation de catalyseurs suivant la revendication 5, **caractérisé en ce que**, comme sels, on met en oeuvre des chlorures.

7. Procédé de préparation de 1,2-dichloroéthane, **caractérisé en ce qu'**on fait réagir de l'éthylène avec du chlorure d'hydrogène et de l'air ou de l'oxygène en présence d'un catalyseur suivant l'une des revendications 1 à 4, à une température de 150 à 400°C et à une pression de 1 à 10 bars.

8. Utilisation de catalyseurs, contenant comme composants actifs
(i) 1 à 15 % en poids de cuivre,
(ii) 0,1 à 6 % en poids de métaux alcalins,
(iii) 0 à 5 % en poids de métaux alcalino-terreux, de métaux des terres rares ou de leurs mélanges,
et un support de catalyseur à quantités de δ-Al₂O₃ détectables par diffractomètre aux rayons X, pour des réactions d'oxychloration catalysées de façon hétérogène.

9. Utilisation de catalyseurs suivant la revendication 8, **caractérisée en ce que**, pour ce qui concerne la réaction d'oxychloration, il s'agit de la conversion d'éthylène en 1,2-dichloroéthane.
